# EUROPÄISCHE PATENTSCHRIFT

(11) **EP 3 618 744 B1**
(45) Veröffentlichungstag und Bekanntmachung des Hinweises auf die Patenterteilung: **08.04.2026**
(21) Anmeldenummer: 18722520.6
(22) Anmeldetag: 04.05.2018
(51) Int. Cl.: A61B 18/12

(54) **GENERATOR FÜR DIE ABGABE HOCHFREQUENTEN WECHSELSTROMS AN EIN MEDIZININSTRUMENT**
GENERATOR FOR THE DELIVERY OF HIGH FREQUENCY ALTERNATING CURRENT TO A MEDICAL INSTRUMENT
GÉNÉRATEUR POUR LA DISTRIBUTION DE COURANT ALTERNATIF HAUTE FRÉQUENCE À UN INSTRUMENT MÉDICAL

(30) Priorität: 04.05.2017 DE 102017109638
(43) Veröffentlichungstag der Anmeldung: 11.03.2020
(73) Patentinhaber: Olympus Winter&Ibe GmbH, 22045 Hamburg (DE)
(72) Erfinder: FÄHSING, Thomas, 38889 Blankenburg (DE)
(74) Vertreter: Eisenführ Speiser
(86) Internationale Anmeldenummer: PCT/EP2018/061551
(87) Internationale Veröffentlichungsnummer: WO 2018/202872

(56) Entgegenhaltungen:
- DE-A1- 3 329 582
- DE-A1- 3 329 582
- DE-A1- 3 427 517
- DE-A1- 3 427 517
- US-A1- 2003 036 757
- US-A1- 2003 036 757
- US-A1- 2005 101 947
- US-A1- 2005 101 947

## Beschreibung

Die Erfindung betrifft einen Generator für die Abgabe hochfrequenten Wechselstroms an ein Medizininstrument, zum Beispiel für das Schneiden und/oder Koagulieren von biologischem Gewebe. Der Generator weist Anschlüsse auf, an die ein entsprechendes Instrument angeschlossen werden kann, um das Instrument elektrisch mit dem Generator zu verbinden und im Betriebsfall mit hochfrequentem Wechselstrom zu versorgen. Generator und Instrument bilden zusammen ein Hochfrequenz- oder HF-Chirurgiegerät.

Hochfrequenz- oder HF-Chirurgiegeräte sind seit langem aus dem Stand der Technik bekannt. Die vom HF-Chirurgiegerät erzeugte und im Anwendungsfall abgegebene HF-Energie wird beispielsweise zum Schneiden oder Koagulieren am menschlichen Körper verwendet. Für die Anwendung wird ein elektrochirurgisches Instrument, mittels dem die HF-Energie in das Gewebe eingeleitet wird, an den Generator angeschlossen. Bei einer monopolaren Anwendung wird auch noch eine separate Neutral- oder Rückleitelektrode angeschlossen, die zur Rückleitung der Energie an das HF-Chirurgiegerät dient. Bei einer bipolaren Anwendung ist die Rückleitelektrode am Instrument angeordnet.

Moderne Generatoren für HF-Chirurgiegeräte, die den aktuellen Sicherheitsanforderungen entsprechen, weisen eine Anwendungseinheit und einen galvanisch davon getrennten Zwischenstromkreis auf. Das elektrochirurgische Instrument wird mit einer Anwendungseinheit des Generators verbunden. Somit steht der Anwendungseinheit bei der elektrochirurgischen Anwendung mit dem Gewebe eines Patienten direkt in Kontakt. Die Anwendungseinheit und der Zwischenstromkreis sind für die Sicherheit des Patienten und des Benutzers galvanisch voneinander getrennt. Ein Beispiel eines solchen HF-Chirurgiegerätes ist in DE 10 2010 025298 A1 beschrieben. Andere HF-Chirurgiegeräte sind in US 2003/0036757 A1, DE 33 29 582 A1, US 2005/0101947 A1 und DE 34 27 517 A1 beschrieben.

Es die Aufgabe der vorliegen Erfindung ein verbessertes HF-Chirurgiegerät bereitzustellen, das eine hohe Betriebssicherheit bietet.

Erfindungsgemäß wird diese Aufgabe durch einen Generator für die Abgabe hochfrequenten Wechselstroms an ein Medizininstrument gelöst, der eine Stromversorgungseinheit, eine Hochfrequenzgeneratorprimäreinheit (HF-Generatorprimäreinheit), eine Anwendungseinheit und eine Steuereinheit aufweist. Dabei ist die HF-Generatorprimäreinheit mit der Stromversorgungseinheit und der Anwendungseinheit verbunden und ausgebildet, die Anwendungseinheit im Betrieb mit hochfrequentem Wechselstrom zu versorgen. Die Anwendungseinheit ist über wenigstens ein Relais mit Anschlüssen zum Anschluss eines Medizininstruments schaltbar elektrisch verbunden. Die Steuereinheit ist galvanisch von der Anwendungseinheit getrennt und ausgebildet, das wenigstens eine Relais und ggf. die Anwendungseinheit zu steuern. Die Steuereinheit ist über einen Zwischensteuerkreis mit der Anwendungseinheit verbunden, wobei der Zwischensteuerkreis sowohl von der Steuereinheit als auch von der Anwendungseinheit galvanisch getrennt ist. Der Zwischensteuerkreis ist ausgebildet, im Betrieb des Generators Steuersignale von der Steuereinheit zu empfangen und in Abhängigkeit dieser Steuersignale Relaissteuersignale zu bilden und an das wenigstens eine Relais der Anwendungseinheit auszugeben.

Ein erfindungsgemäßer Zwischensteuerkreis bietet den Vorteil, dass die Relaisspulen sowie die Rücklesekontakte der von dem Zwischensteuerkreis angesteuerten Relais mit wesentlich geringen Isolationsabständen ausgeführten werden können. Dadurch sind beispielsweise Relais kleinerer Bauweise möglich. Dies führt zur Platz- sowie Gewichtseinsparungen. Der Grund für die möglichen geringeren Isolationsabstände ist der, dass der Zwischensteuerkreis so gestaltet werden kann, dass die maximale Potenzialdifferenz zwischen der Anwendungseinheit und dem Zwischensteuerkreis - und damit die Isolationsspannung, für die die Relais ausgelegt sein müssen - geringer sein kann, wenn der Zwischensteuerkreis galvanisch von der Steuereinheit des Generators getrennt ist, so dass bereits zwischen dem Zwischenkreis des Generators und dem Zwischensteuerkreis eine größere maximale Potentialdifferenz herrschen darf. An dieser Schnittstelle führt eine solche höhere maximale Potenzialdifferenz zu einem geringeren Aufwand für das Erzielen einer entsprechend hohen Isolationsspannung.

HF-Primäreinheit und Steuereinheit können auf einer Platine verwirklicht sein und eine Primär-Versorgungs- und Steuereinheit bilden. Der Zwischensteuerkreis bildet dann eine von dieser galvanisch getrennten Sekundär-Steuereinheit, die die Ausgänge der Anwendungseinheit schaltenden Relais ansteuert und ausliest.

Vorzugsweise erfolgt die Spannungsversorgung des Zwischensteuerkreises durch zwischen dem Zwischensteuerkreis und der Steuereinheit angeordnete Übertrager, beispielsweise Gleichstromübertrager wie beispielsweise DC/DC-Konverter.

Zur Signalübermittlung von der Steuereinheit zu dem Zwischensteuerkreis sind vorzugsweise solche Übertrager wie Optokoppler vorgesehen.

Der Zwischensteuerkreis weist vorzugsweise eine Relaissteuereinheit auf, beispielsweise einen Controller, der auf der einen Seite mit dem wenigstens einem Relais verbunden ist, um dieses auszusteuern und dessen Rücklesekontakte auszulesen. Auf der anderen Seite ist die Relaissteuereinheit über Übertrager, beispielsweise die Optokoppler, mit der Steuereinheit verbunden und kann auf diese Weise mit der Steuereinheit Steuersignale austauschen.

Da die Relaissteuereinheit als Controller ausgeführt sein kann, können weniger Signalübertrager, beispielweise weniger Optokoppler, zwischen dem Zwischensteuerkreis und der Steuereinheit vorgesehen sein, als Relais anzusteuern sind, da über die Signalübertrager auch komplexere, kodierte Signale übertragen werden können, die von der Relaissteuereinheit dekodiert und in Relaissteuersignale für mehrere Relais umgesetzt werden können.

Auch dies trägt dazu bei, dass zwischen Steuereinheit und Zwischensteuerkreis nur wenige hochspannungsfeste Bauteile wie Optokoppler oder DCDC-Konverter vorgesehen werden müssen. Damit ist es leichter möglich, zwischen der Steuereinheit und dem Zwischensteuerkreis eine Isolationsspannung von mehr als 2 kV zu realisieren, beispielsweise mehr als 4 kV zu realisieren. Dies erlaubt es auf der anderen Seite, dass die Isolationsspannung zwischen dem Zwischensteuerkreis und der Anwendungseinheit auf maximal 1 kV beschränkt sein kann, so dass die Relais auch nur für diese Isolationsspannung auslegt zu sein brauchen.

Die Anwendungseinheit des Generators weist vorzugsweise eine Hochfrequenzgeneratorschaltung auf, die mit der HF-Generatorprimäreinheit einen Hochspannungstransformator bildet, so dass die Anwendungseinheit von der HF-Generatorprimäreinheit ebenfalls galvanisch getrennt ist. Dies erlaubt es wieder, die HF-Generatorprimäreinheit und die Steuereinheit auf einem gemeinsamen Potentialniveau zu betreiben, so dass die Steuereinheit und die HF-Generatorprimäreinheit nicht galvanisch voneinander getrennt zu sein brauchen.

Die Erfindung soll nun anhand eines Ausführungsbeispiels mit Bezug auf die Figuren näher erläutert werden. Von den Figuren zeigt
- Figur 1: ein schematisches Blockschaltbild eines erfindungsgemäßen Generators, und

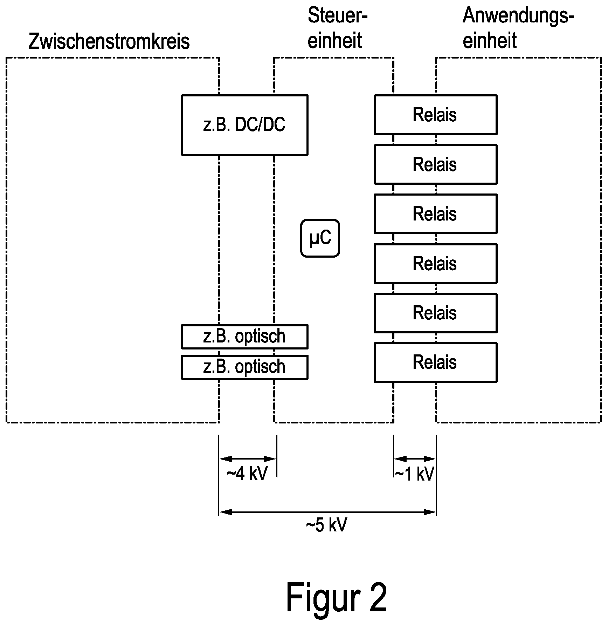

- Figur 2: eine zweite, schematische Darstellung des erfindungsgemäßen Generators.

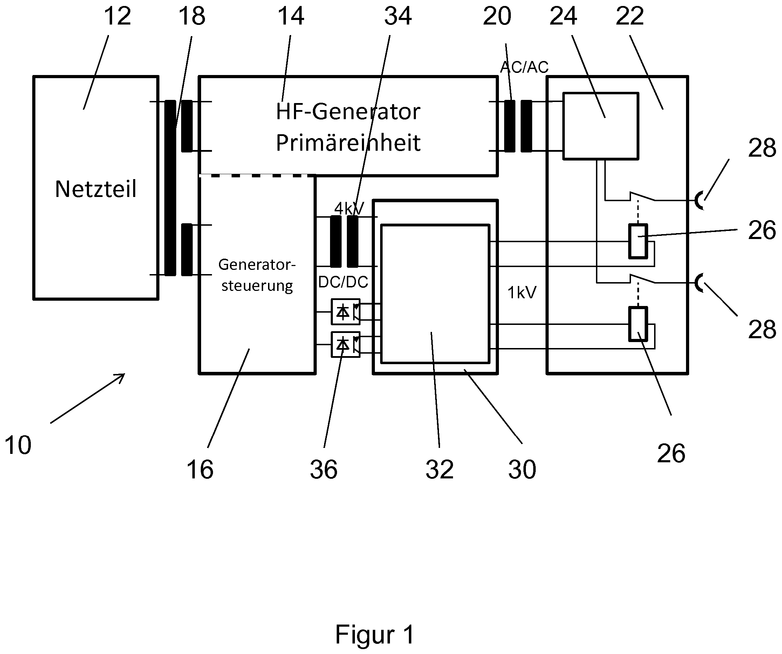

Der in Figur 1 abgebildete Generator 10 besitzt eine Stromversorgungseinheit 12, das mit einer HF-Generatorprimäreinheit 14 sowie einer Steuereinheit 16 des Generators 10 verbunden ist und diese Bestandteile des Generators mit Energie versorgt. Beispielsweise können die HF-Generatorprimäreinheit 14 und die Steuereinheit 16 über einen Transformator 18 mit der Stromversorgungseinheit 12 verbunden sein. Die Stromversorgungseinheit 12 kann aber auch eine Batterienetzteil sein oder eine Art von Stromversorgungseinheit.

Teil der HF-Generatorprimäreinheit 16 ist eine Primärspule eines Hochfrequenztransformators 20, dessen Sekundärseite Teil einer Anwendungseinheit 22 ist. Die Anwendungseinheit 22 weist eine Hochfrequenzgeneratorschaltung 24 auf, die ausgebildet ist, eine hochfrequente Hochspannung zu generieren, die beispielsweise an ein im Betrieb des Generators 10 an ein an diesen angeschlossenes Medizininstrument abgegeben werden kann. Dazu ist die Hochfrequenzgeneratorschaltung 24 über Relais 26 mit Anschlüssen 28 zum Anschließen eines oder mehrerer Medizininstrumente verbunden. Über die Relais 26 ist die Verbindung zwischen der Hochfrequenzgeneratorschaltung 24 und den Anschlüssen 28 schaltbar.

Die Ansteuerung der Relais 26 sowie das Auslesen von deren Auslesekontakten erfolgt durch einen Zwischensteuerkreis 30, der eine Relaissteuereinheit 32 aufweist. Der Zwischensteuerkreis 30 ist zum einen mit der Steuereinheit 16 verbunden, und zwar einerseits über einen DC/DC-Konverter 34 und andererseits über Optokoppler 36. Dadurch ist der Zwischensteuerkreis 30 galvanisch von der Steuereinheit 16 getrennt. Der DC/DC-Konverter 34 dient dazu, den Zwischensteuerkreis 30 mit Energie zu versorgen. Die Optokoppler 36 dienen dazu, Steuersignale zwischen der Steuereinheit 14 und der Relaissteuereinheit 32 des Zwischensteuerkreises 30 zu übertragen.

Die Relaissteuereinheit 32 ist ausgebildet seitens der Steuereinheit 16 über die Optokoppler 36 empfangene Steuersignale in Relaissteuersignale umzusetzen, mit denen die Relais 26 einzeln angesteuert werden können. Außerdem kann die Relaissteuereinheit 32 die Rücklesekontakte der Relais 26 auslesen und entsprechende Kontrollsignale generieren, die die Relaissteuereinheit 32 über die Optokoppler 36 an die Steuereinheit 16 übermitteln kann. Die Optokoppler 36 sowie der DC/DC-Konverter 34 wie auch die entsprechenden Abstände zwischen Zwischensteuerkreis 30 und Steuereinheit 16 sind so gewählt, dass die Isolationsspannung zwischen der Steuereinheit 16 und dem Zwischensteuerkreis 30 beispielsweise 4 kV beträgt. Auf der anderen Seiten braucht die Isolationsspannung zwischen dem Zwischensteuerkreis 30 und den Anwendungsstromkreis 22 nur beispielsweise 1 kV zu betragen, so dass es ausreicht, die Relais 26 für eine Isolationsspannung von 1 kV zu dimensionieren und nicht etwas für die maximale Ausgangsspannung des Generators 10 von beispielsweise 5 kV.

### Bezugszeichenliste

- 10: Generator
- 12: Stromversorgungseinheit
- 14: Hochfrequenzgeneratorprimäreinheit (HF-Generatorprimäreinheit)
- 16: Steuereinheit
- 18: Transformator
- 20: Hochfrequenztransformator
- 22: Anwendungseinheit
- 24: Hochfrequenzgeneratorschaltung
- 26: Relais
- 28: Anschlüsse
- 30: Zwischensteuerkreis
- 32: Relaissteuereinheit
- 34: DC/DC-Konverter
- 36: Optokoppler

## Patentansprüche

1. Generator (10) für die Abgabe hochfrequenten Wechselstroms an ein Medizininstrument, mit einer Stromversorgungseinheit (12), einer HF-Generatorprimäreinheit (14), einer Anwendungseinheit (22) und einem Steuereinheit (16) , von denen die HF-Generatorprimäreinheit (14) mit der Stromversorgungseinheit (12) und der Anwendungseinheit (22) verbunden und ausgebildet ist, die Anwendungseinheit (22) im Betrieb mit hochfrequentem Wechselstrom zu versorgen, wobei die Anwendungseinheit (22) über wenigstens ein Relais (26) mit Anschlüssen (28) zum Anschluss eines Medizininstruments schaltbar elektrisch verbunden ist und wobei die Steuereinheit (16) galvanisch von der Anwendungseinheit (22) getrennt und ausgebildet ist, das wenigstens eine Relais (26) und ggf. die Anwendungseinheit (22) zu steuern,
wobei die Steuereinheit (16) über einen Zwischensteuerkreis (30) mit der Anwendungseinheit (22) verbunden ist, wobei der Zwischensteuerkreis (30) sowohl von der Steuereinheit (16) als auch von der Anwendungseinheit (22) galvanisch getrennt ist und **dadurch gekennzeichnet ist, dass**
der Zwischensteuerkreis (30) ausgebildet ist, im Betrieb des Generators (10) Steuersignale von der Steuereinheit (16) zu empfangen und in Abhängigkeit dieser Steuersignale Relaissteuersignale zu bilden und an das wenigstens eine Relais (26) der Anwendungseinheit (22) auszugeben.

2. Generator (10) gemäß Anspruch 1, **dadurch gekennzeichnet, dass** der Zwischensteuerkreis (30) über wenigstens einen Optokoppler (36) mit der Steuereinheit (16) verbunden ist, wobei der Optokoppler (36) dazu ausgebildet ist, Steuersignale von der Steuereinheit (16) zu dem Zwischensteuerkreis (30) zu übertragen, und wobei der Zwischensteuerkreis (30) eine Relaissteuereinheit (32) aufweist, die ausgebildet ist, von der Steuereinheit (16) empfangene Steuersignale zu verarbeiten und in Abhängigkeit der empfangenen Steuersignale Relaissteuersignale zu generieren.

3. Generator (10) gemäß Anspruch 2, **dadurch gekennzeichnet, dass** die Anwendungseinheit (22) mehrere Relais (26) aufweist und die Anzahl der Optokoppler (36) zwischen Steuereinheit (16) und Zwischensteuerkreis (30) kleiner ist, als die Anzahl der Relais (26).

4. Generator (10) nach mindestens einem der vorangehenden Ansprüche, **dadurch gekennzeichnet, dass** eine Isolationsspannung zwischen Steuereinheit (16) und Zwischensteuerkreis (30) größer ist als 2 Kilovolt, während eine Isolationsspannung zwischen Zwischensteuerkreis (30) und Anwendungseinheit (22) maximal 1 kV beträgt.

5. Generator (10) nach mindestens einem der vorangehenden Ansprüche, **dadurch gekennzeichnet, dass** die Anwendungseinheit (22) eine Hochfrequenzgeneratorschaltung (24) aufweist, die mit der HF-Generatorprimäreinheit (14) verbunden und ausgebildet ist, im Betrieb an ein angeschlossenes Medizininstrument eine hochfrequente Hochspannung abzugeben, wobei die Hochfrequenzgeneratorschaltung (24) über das wenigstens eine Relais (26) mit den Anschlüssen (28) zum Anschluss eines Medizininstruments schaltbar elektrisch verbunden ist.

## Claims

1. A generator (10) for the delivery of high frequency alternating current to a medical instrument, the generator having a power supply unit (12), an HF generator primary unit (14), an application unit (22) and a control unit (16), wherein the HF generator primary unit (14) is connected to the power supply unit (12) and the application unit (22) and designed to supply the application unit (22) with high frequency alternating current during operation, wherein the application unit (22) via at least one relay (26) is electrically connected in a switchable manner to terminals (28) for connecting a medical instrument, and wherein the control unit (16) is galvanically separated from the application unit (22) and designed to control the at least one relay (26) and the application unit (22) as applicable,
**characterized in that** the control unit (16) is connected to the application unit (22) via an intermediate control circuit (30), wherein the intermediate control circuit (30) is galvanically separated both from the control unit (16) and from the application unit (22) and the intermediate control circuit (30) is designed to receive during the operation of the generator (10) control signals from the control unit (16) and to generate relay control signals in dependence on said control signals and to output the relay control signals to the at least one relay (26) of the application unit (22).

2. A generator (10) pursuant to claim 1, **characterized in that** the intermediate control circuit (30) is connected to the control unit (16) via at least one optocoupler (36), wherein the optocoupler (36) is designed to transmit control signals from the control unit (16) to the intermediate control circuit (30), and wherein the intermediate control circuit (30) has a relay control unit (32) that is designed to process the control signals received from the control unit (16) and to generate relay control signals in dependence on the received control signals.

3. A generator (10) pursuant to claim 2, **characterized in that** the application unit (22) has several relays (26) and that the number of optocouplers (36) between the control unit (16) and the intermediate control circuit (30) is smaller than the number of relays (26).

4. A generator (10) pursuant to at least one of the previous claims, **characterized in that** an isolation voltage between the control unit (16) and the intermediate control circuit (30) is higher than 2 kilovolt, while the isolation voltage between the intermediate control circuit (30) and the application unit (22) is no higher than 1 kV.

5. A generator (10) pursuant to at least one of the previous claims, **characterized in that** the application unit (22) has a high frequency generator circuit (24) that is connected to the HF generator primary unit (14) and designed to supply a connected medical instrument with high frequency high voltage during operation, wherein the high frequency generator circuit (24) is electrically connected in a switchable manner to the terminals (28) for connecting a medical instrument via the at least one relay (26).

## Revendications

1. Générateur (10) de distribution de courants alternatifs de haute fréquence à un instrument médical, comprenant une unité (12) d'alimentation en courant, une unité (14) primaire de générateur HF, une unité (22) d'application et une unité (16) de commande, dont l'unité (14) primaire de générateur HF est raccordée à l'unité (12) d'alimentation en courant et à l'unité (22) d'application et est constituée pour alimenter en fonctionnement en courant alternatif de haute fréquence l'unité (22) d'application, dans lequel l'unité (22) d'application peut être connectée électriquement par l'intermédiaire d'au moins un relais (26) à des bornes (28) pour le raccordement d'un instrument médical et dans lequel l'unité (16) de commande est séparée galvaniquement de l'unité (22) d'application et est constituée pour commander le au moins un relais (26) et éventuellement l'unité (22) d'application,
dans lequel l'unité (16) de commande est raccordée à l'unité (22) d'application par un circuit (30) de commande intermédiaire, dans lequel le circuit (30) de commande intermédiaire est séparé galvaniquement, tant de l'unité (16) de commande qu'également de l'unité (22) d'application, et **caractérisé en ce que** le circuit (30) de commande intermédiaire est constitué pour recevoir, lorsque le générateur (10) fonctionne, des signaux de commande de l'unité (16) de commande et pour former, en fonction de ces signaux de commande, des signaux de commande de relais et les donner au au moins un relais (26) de l'unité (22) d'application.

2. Générateur (10) suivant la revendication 1, **caractérisé en ce que** le circuit (30) de commande intermédiaire est raccordé à l'unité (16) de commande par l'intermédiaire d'au moins un optocoupleur (36), dans lequel l'optocoupleur (36) est constitué pour transmettre des signaux de commande de l'unité (16) de commande au circuit (30) de commande intermédiaire, et dans lequel le circuit (30) de commande intermédiaire a une unité (32) de commande de relais, qui est constituée pour traiter des signaux de commande reçus de l'unité (16) de commande et créer des signaux de commande de relais en fonction des signaux de commande reçus.

3. Générateur (10) suivant la revendication 2, **caractérisé en ce que** l'unité (22) d'application a plusieurs relais (26) et le nombre des optocoupleurs (36), entre l'unité (16) de commande et le circuit (30) de commande intermédiaire, est plus petit que le nombre des relais (26).

4. Générateur (10) suivant au moins l'une des revendications précédentes, **caractérisé en ce qu'**une tension d'isolation, entre l'unité (16) de commande et le circuit (30) de commande intermédiaire, est plus grande que 2 kilovolt, tandis qu'une tension d'isolation, entre le circuit (30) de commande intermédiaire et l'unité (22) d'application, est de 1 kV au maximum.

5. Générateur (10) suivant au moins l'une des revendications précédentes, **caractérisé en ce que** l'unité (22) d'application a un circuit (24) de générateur de haute fréquence, qui est raccordé à l'unité (14) primaire de générateur HF et constitué pour donner, en fonctionnement, une haute tension de haute fréquence à un instrument médical raccordé, dans lequel le circuit (24) de générateur de haute fréquence peut, par l'intermédiaire d'au moins un relais (26), être connecté aux bornes (28) pour le raccordement d'un instrument médical.
